# FASCICULE DE BREVET EUROPEEN

(11) **EP 2 575 910 B1**
(45) Date de publication et mention de la délivrance du brevet: **26.07.2017**
(21) Numéro de dépôt: 11728327.5
(22) Date de dépôt: 30.05.2011
(51) Int. Cl.: A61L 27/18, A61L 27/24

(54) **MATRICES DENSES DE COLLAGENE POUR LA REPARATION TISSULAIRE ET LEUR PROCEDE DE PREPARATION**
DICHTE FASERIGE KOLLAGENMATRIZEN FÜR GEWEBEREPARATUR UND VERFAHREN ZU IHRER HERSTELLUNG
DENSE FIBRILLAR COLLAGEN MATRICES FOR TISSUE REPAIR AND THE PREPARATION METHOD THEREOF

(30) Priorité: 31.05.2010 FR 1054194
(43) Date de publication de la demande: 10.04.2013
(73) Titulaire: Centre National de la Recherche Scientifique (C.N.R.S.), 75016 Paris (FR); Université de Versailles Saint-Quentin-en-Yvelines, 78035 Versailles Cédex (FR); Université Pierre et Marie Curie (Paris 6), 75005 Paris (FR)
(72) Inventeur: GIRAUD-GUILLE, Marie-Madeleine, F-75012 Paris (FR); NASSIF, Nadine, F-75005 Paris (FR); WANG, Yan, F-75015 Paris (FR); HELARY, Christophe, F-94500 Champigny sur Marne (FR); PELLE, Anne, F-92170 Vanves (FR)
(74) Mandataire: Bourgouin, André
(86) Numéro de dépôt international: PCT/FR2011/051234
(87) Numéro de publication internationale: WO 2011/151587

(56) Documents cités:
- EP-A2- 0 457 430
- WO-A1-2006/029571
- US-A1- 2009 054 350

## Description

La présente invention a pour objet un procédé de préparation de matrices fibrillaires denses de collagène, les matrices ainsi obtenues et leurs utilisations pour la réparation tissulaire.

### I- ETAT DES LIEUX ET ATTENTES :

Les enjeux économiques et sociétaux des biomatériaux sont considérables du fait de l'allongement significatif de la durée de vie dans les pays occidentaux et des pathologies qui y sont associées.

De nombreux matériaux de substitution, de nature synthétique (métaux, céramiques, polymères) ou naturelle (chitosane, dextrane, pullulane), glycoaminoglycanes (GAG) ou collagène) sont proposés dans la littérature. Les critères de qualité des biomatériaux sont déterminés par leur facilité d'utilisation, leurs propriétés mécaniques et leur devenir dans l'organisme (rejet ou dégradation, adhésion et colonisation cellulaire, remodelage).

Ainsi dans le contexte actuel, différents types de substituts à base de collagène sont déjà proposés au niveau d'applications industrielles, ils restent cependant encore imparfaits pour répondre pleinement aux attentes des chirurgiens et des patients.

Les premiers biomatériaux à base de collagène ayant conduit à des applications industrielles, ont concerné les substituts de peau. Les produits commercialisés ont été proposés comme implants chez les grands brûlés ou pansements au niveau de blessures chroniques et d'ulcères. Le premier substitut dermique, vendu sous le nom d'Integra ® est une éponge acellulaire de collagène et protéoglycanes pontée au glutaraldéhyde. Associé à un feuillet de kératinocytes, commercialisé sous le nom d'Epicel ®, le derme équivalent devient un substitut de peau.

Dans d'autres applications les substituts incluent des polymères d'origine synthétique ou intègrent des cellules et leur succès va dépendre des interrelations cellules/matrices. L'adhésion des cellules dans de larges pores, au sein d'éponges lyophillisées de collagène (Orcel ® ou Integra ®) les placent dans une situation où le support est en 2D, dans ce cas le phénotype diffère de la situation du *in vivo* où les cellules ont des contacts en 3D. Cela renforce l'avantage des molécules d'origine naturelle, en particulier le collagène, pour favoriser l'adhésion et la colonisation cellulaire. Les seuls systèmes où les fibroblastes se retrouvent avec des contacts en 3D sont les hydrogels de collagène développés aux Etats-Unis. Ces hydrogels associés à des feuillets de kératinocytes deviennent alors des matériaux de réparation cutanée (Apligraf ®). L'inconvénient majeur de ces biomatériaux réside en leurs mauvaises propriétés mécaniques.

Les matériaux à base de collagène actuellement implantables dérivent de deux procédés de fabrication, conduisant soit à des éponges, soit à des hydrogels. Ces matériaux de base subissent ensuite des traitements venant renforcer la qualité des produits commerciaux par association avec des molécules chimiques (consolidation), des cellules (fibroblastes) ou des films protecteurs (silicone). Les éponges de collagène, obtenues par lyophilisation de solutions acido-solubles de collagène sont des systèmes tridimensionnels poreux. La taille des pores, de 15 à 150 µm de diamètre, varie selon les critères de congélation précédant la lyophilisation. La structure en éponge permet aux fibroblastes ensemencés de proliférer en colonisant entièrement le substrat puis de sécréter du collagène fibrillaire. Les applications, en thérapie cellulaire ou génique, de ces systèmes sont liées à leurs propriétés de support biocompatible et biodégradable. Toutefois la fragilité des éponges et leur faible durée de vie dans l'organisme nécessite une réticulation par des agents chimiques (aldéhydes, carbodiimides) dont la toxicité pose problèmes. Les hydrogels de collagène sont obtenus par précipitation des monomères de collagène en fibrilles au sein d'une solution acido-soluble. Lorsque des fibroblastes sont associés à la solution de départ et à du milieu de culture, le gel fibrillaire faiblement concentré (∼ 1mg/ml) et de structure aléatoire, emprisonne les cellules. Une contraction importante du gel s'observe alors dans les jours qui suivent (∼ 3% de surface initiale après 14 jours de culture) et aboutit à ce que l'on appelle un derme équivalent. Ce tissu reconstitué devient une peau équivalente lorsqu'il est secondairement ensemencé par des cellules de l'épiderme, les kératinocytes, qui prolifèrent et recouvrent la structure. Bien que les essais de greffes chez l'humain aient montré les qualités biologiques de ces substituts, les phénomènes de contraction du réseau fibrillaire lâche pose toutefois des problèmes de fragilité évidents, pendant la manipulation des matériaux ou après leur implantation. Ces hydrogels ont fait l'objet de nombreuses études en termes de remodelage (synthèse de collagène et de métalloprotéinases) et de prolifération cellulaire. Alors que les hydrogels sont d'intéressants modèles de culture cellulaire, ils se sont avérés insuffisants en tant que substrats pour la réparation tissulaire. Les propriétés mécaniques des hydrogels ont été améliorées en augmentant leur concentration par compression (cf. §II.4), aboutissant alors à des films facilement déshydratés et friables, leur épaisseur de l'ordre de 40 µm est trop fine pour beaucoup d'applications.

En parallèle, des tissus biologiques décellularisés d'origine animale (valves cardiaques, vaisseaux, dermes, tissus de soutien d'origine bovine ou porcine), associés à d'autres composants, ont été mis sur le marché. Dans ce type d'approche, des complications, liées aux modes de préparation (débris cellulaires résiduels ou cytotoxicité liée aux agents réticulants) font état de réactions inflammatoires importantes et ont des conséquences graves en terme de morbidité et mortalité.

Aussi existe-t-il un besoin de nouveaux biomatériaux qui répondent mieux aux attentes du monde médical et des patients, notamment des biomatériaux dépourvus de toxicité, de formes variables ou en recouvrement de matériaux, colonisables par les cellules et intégrables par les tissus de l'hôte. Le brevet WO2006/29571 divulgue une méthode pour former une matrice de collagène avec des propriétés mécaniques améliorées. Le pH d'une solution de collagène est augmenté afin de former des fibrilles. puis le gel est mis en contact avec un agent de réticulation. La formation de liaisons covalentes au sein du gel de collagène va agir sur sa stabilité et ses propriétés mécaniques vont être améliorées. Le document EP 0457430 divulgue la préparation d'un gel de collagène par dialyse contre une solution de polyéthylène glycol. Les inventeurs ont développé des techniques permettant de travailler avec du collagène à des concentrations élevées, en contrôlant la mise en place d'un ordre supramoléculaire lié à la concentration et en stabilisant lors d'une transition sol/gel pour aboutir à des matrices fibrillaires denses. Les inventeurs ont également montré que des matrices à 40 mg/ml de collagène présentent des propriétés particulièrement intéressantes au niveau de leurs réponses cellulaire et mécanique. Elles sont colonisées *in vitro* par des fibroblastes ensemencés en leur surface suite à l'hydrolyse du collagène *via* des métalloprotéases. Après 28 jours de culture le nombre de cellules présentes au sein de la matrice, résultant d'une balance entre prolifération et apoptose, est proche de celui d'un tissu vivant (Helary et al. Biomaterials, (2005), 26 p. 1533-1543 ; Helary et al. Biomaterials (2006) 27: 4443-4454. Les réponses mécaniques sous compression uniaxiale donnent des résultats équivalents à des échantillons témoins de derme de rat suggérant que ces matériaux pourraient être utilisés en réparation tissulaire (Ramtani et al. JMMB, 2010).

La demande WO 2010/004182 au nom des inventeurs concerne la minéralisation de ces matrices fibrillaires denses de collagène dans une gamme de concentration plus élevée (à partir de 80 mg/mL) pour une application dans le domaine des substituts osseux.

Dans les travaux précédemment utilisés, les inventeurs ont utilisé différents procédés pour concentrer le collagène avec comme objectif l'obtention d'une matrice fibrillaire dense de collagène.

### II- LES PRECEDENTS PROCEDES D'OBTENTION DE MATRICES DENSES :

### 1) L'évaporation

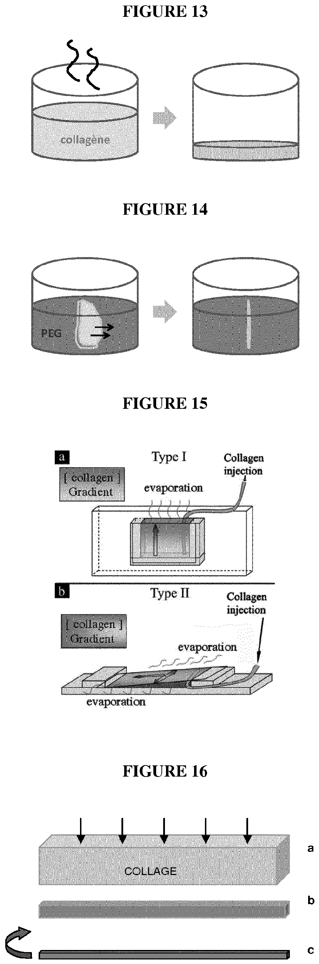

*Procédé -* Les solutions acides initiales de collagène, dont la concentration initiale est inférieure à 5 mg/mL, sont placées dans un cristallisoir (ou tout autre récipient large ouvert) sous une hotte en milieu stérile. On laisse évaporer la solution jusqu'à la concentration souhaitée (Hélary et al. Biomaterials, 2005 ; figure 13).

*Limites -* Cette technique n'est pas adaptée pour de très grandes concentrations et de grand volume. En effet si on part d'une solution initiale de 1 mg/mL pour obtenir une solution à 300 mg/mL, il faut un récipient très grand et à priori très large afin de ne pas attendre un temps conséquent pour récupérer son échantillon. De plus un film de collagène sec peut se former en surface. En outre après précipitation par remontée de pH, les fibrilles de collagène ne sont pas toujours homogènes en taille. Cette technique est peu adaptable/transférable à l'industrie car il faudrait une zone de stérilité importante du fait de l'utilisation d'une cuve ouverte. En outre, à de très fortes concentrations ou à plus forte raison pour de grands volumes, un gradient de concentration se forme (zone plus concentrée à la surface c'est-à-dire à l'interface air/solution) et il semble difficile d'atteindre l'équilibre. L'existence de ce gradient explique en partie que les fibrilles de collagène ne soient pas toujours homogènes en taille.

### 2) La dialyse

*Procédé -* Les solutions acides de collagène dont la concentration initiale est inférieure à 5 mg/mL sont placées dans un boudin de dialyse. La porosité de la membrane est fixée pour que le solvant, les sels et les molécules dont le poids moléculaire est inférieur à la taille des pores diffusent à travers la paroi mais pas le collagène. L'ensemble est immergé dans une solution de polymère (en général du polyéthylène glycol ou PEG) dont la concentration est égale/ou un peu supérieure à celle que l'on veut atteindre (Gobeaux et al. J. Mol. Biol. 2008 ; figure 14).

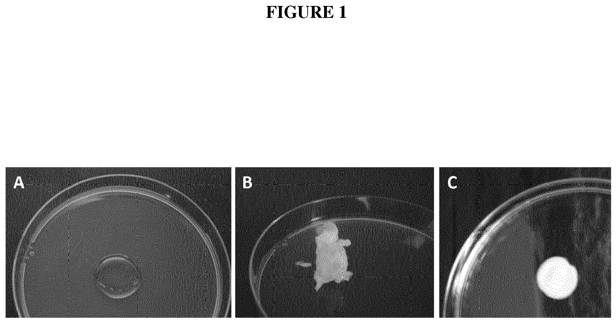

*Limites -* Avec cette technique, si on part d'une solution initiale de 1 mg/mL pour obtenir une solution à 300 mg/mL, il faut un volume de membrane au moins égal à 300 mL pour obtenir un volume final de 1 mL de solution concentrée, ce qui expérimentalement n'est pas évident. Aussi en général, utilise-t-on des volumes de membranes de dialyse moins importants et on recharge en collagène régulièrement jusqu'à obtenir un volume de solution assez conséquent pour que l'on puisse l'extraire. Cette étape pose également problème car il faut alors « gratter » le collagène concentré réparti un peu partout à l'intérieur de la membrane, ce qui risque, du fait du cisaillement, de modifier l'organisation du collagène qui à cette concentration possède des propriétés d'organisation de type cristal-liquide. De ce fait l'organisation finale est difficilement contrôlable. A cause de cela, après précipitation par remontée de pH, les fibrilles de collagène ne sont pas homogènes en taille. De plus, la solution concentrée de collagène récupérée n'est pas homogène macroscopiquement (voir figure 1B) donc les propriétés mécaniques du matériau final ne sont pas constantes pour l'ensemble du matériau.

### 3) Les microcellules

*Procédé -* Les solutions acides initiales de collagène sont injectées et concentrées progressivement dans des microchambres en verre fabriquées au laboratoire. Le principe est que l'on injecte entre lame et lamelle du collagène dilué qui en diffusant vers les bords de la microchambre à l'interface avec l'air, se concentre petit à petit par évaporation de la solution. Plus on va vers les bords de la microchambre (vers l'interface air/solution) plus le collagène est concentré. Un gradient de concentration très large du collagène (∼5 à 1000 mg/mL) se forme au sein de la solution finale concentrée (G. Mosser et al., Matrix Biol. 2006 ; figure 15).

*Limites -* Il n'y a donc pas ici de contrôle de la concentration. Ce procédé est intéressant pour étudier des diagrammes de phases et est adapté à des petits volumes (inférieurs à 0,5 mL). Toutefois, la première couche à l'interface air/solution est en réalité une couche diluée car le collagène n'a pas le temps de se concentrer, donc de s'organiser, contrairement à la couche suivante. Après précipitation par remontée de pH, les fibrilles de collagène ne sont donc pas ici homogènes en taille.

### 4) La compression

*Procédé -* Cette méthode propose la compression de gels fibrillaires hydratés de collagène pour obtenir des matrices denses. Les réseaux fibrillaires restent isotropes mais les propriétés mécaniques sont bien supérieures à celles du gel initial. La viabilité de cellules insérées au sein de telles matrices, sous forme de feuillets enroulés, est vérifiée (Brown *et al.* 2005 ; figure 16).

*Limites -* La méthode est ultra rapide et les concentrations en collagène peuvent atteindre 170 mg/mL. Cependant l'hydrogel subit de forts cisaillement et est, de part sa mise en oeuvre, extrêmement déshydraté ce qui le rend fragile et sujet à des infections une fois implanté.

Les limites de ces précédents procédés montre l'intérêt majeur, pour des applications en ingénierie tissulaire, de disposer d'une méthodologie qui permette d'obtenir des matrices de collagène homogènes macroscopiquement ainsi qu'en concentration, en diamètre de fibrilles et dont l'ordre soit contrôlé.

### III- NOUVEAU PROCEDE POUR L'OBTENTION DE MATRICES DE COLLAGENE DENSES ET HOMOGENES

Les inventeurs ont ainsi mis au point un procédé permettant d'obtenir des matrices fibrillaires denses de collagène obtenues à des concentrations 5 à 60 fois plus élevées que les hydrogels classiques, voire plus et ne contenant aucun additif visant à renforcer les propriétés mécaniques mais seulement du collagène pur reconstitué sous forme de fibrilles et de réseaux de fibrilles denses, ce qui évite les phénomènes d'inflammations, de toxicité et de rejets. Les matrices obtenues sont colonisables par des cellules du tissu conjonctif et leurs propriétés mécaniques sont proches de celles de tissus biologiques.

Aussi la présente invention a-t-elle pour objet un procédé de préparation d'un matériau homogène à base de collagène démarrant par la concentration d'une solution de collagène acido-soluble, ledit procédé comprenant :
a) la mise en contact, par injection continue d'une solution de collagène acido-soluble par des moyens de pression contrôlée, avec un élément perméable, ledit élément perméable étant lui-même en contact avec un agent concentrant, plus particulièrement à savoir une solution de polymère,
b) le maintien en contact de la solution de collagène, de l'élément perméable et de la solution de polymère, dans des conditions permettant le transfert sélectif de masse du solvant contenu dans la solution aqueuse de collagène pour obtenir la formation d'une solution plus concentrée de collagène homogène à l'intérieur de l'élément perméable ou à la surface de l'élément perméable.

Conformément à l'invention, l'élément perméable est choisi avec une taille de pores de poids moléculaire (PM) inférieur à celui du collagène d'une part et du polymère externe d'autre part. Le maintien en contact de la solution de collagène séparée de la solution de polymère par l'élément perméable, permet au solvant de diffuser de la solution de collagène interne vers la solution de polymère externe. Cela permet d'obtenir la formation d'une solution plus concentrée de collagène homogène à l'intérieur de l'élément perméable ou à la surface de l'élément perméable par ce transfert sélectif de solvant. Dans un mode de réalisation avantageux de l'invention, pour permettre le transfert sélectif de masse du solvant contenu dans la solution aqueuse de collagène, la pression osmotique de la solution de polymère est supérieure à celle de al solution de collagène.

L'injection continue par des moyens contrôlés peut être réalisée par tout système connu de l'homme du métier susceptible de permettre ce type d'injection, notamment un pousse-seringue électrique ou une pompe. La vitesse d'injection est adaptée pour que la force de pression des deux côtés de la membrane soit identique ; ainsi le flux est adapté en fonction du polymère et de sa viscosité de manière à ce que l'homme du métier trouve la vitesse évitant la rétractation de la membrane ou son gonflement jusqu'à percement. La vitesse dépend du type de polymère utilisé et de son poids moléculaire, lequel est supérieur aux pores de la membrane. L'adaptation de la vitesse relève des connaissances générales de l'homme du métier. A titre d'exemple avec du PEG d'un poids moléculaire de 35 KDa, on peut utiliser une vitesse comprise entre 1 et 15 µl/min. ce qui permet de terminer la formation du matériau au bout de 15 jours pour 28 mL injecté d'une solution à 1 mg/mL. L'injection peut être interrompue pour atteindre l'équilibre (c'est-à dire quand la concentration en collagène est alors homogène partout dans le moule) plus vite donc diminuer le gradient de concentration plus vite et reprise afin d'élaborer un système multi-couches de concentration contrôlée et d'organisation modulable. Il est également possible d'interrompre l'injection sans attendre l'équilibre ce qui permettra d'avoir un gradient continu en concentration.

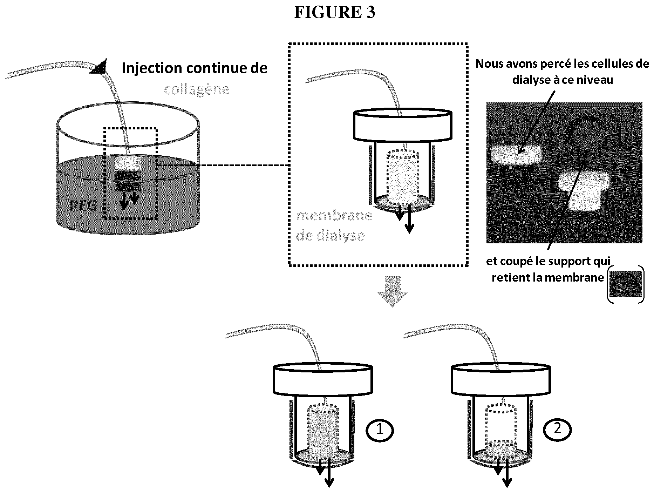

Un exemple de dispositif utilisable pour mettre en oeuvre le procédé de l'invention est illustré dans la figure 3.

Dans un mode de réalisation avantageux du procédé de l'invention, l'élément perméable est une cellule de dialyse et ledit procédé comprend les étapes suivantes :
a) préparation d'une solution acide de collagène pur dans un solvant aqueux,
b) injection continue de la solution de collagène pur par des moyens de pression contrôlée, dans des cellules de dialyse dont au moins une des extrémités est fermée par une membrane de dialyse dont la porosité est fixée pour que seuls le solvant ainsi que l'acide et d'éventuels additifs (tels que des collagènes de différents types, les ions, les précurseurs d'une phase minérale (sels de calcium, phosphate, etc...), des glycosaminoglycanes ou des molécules organiques), s'ils sont présents, diffusent à travers ladite membrane, ladite membrane étant en contact avec une solution de polymère, dont la concentration est adaptée à la concentration finale en collagène,
c) maintien de la solution de collagène, de la cellule de dialyse et du polymère dans des conditions permettant le transfert sélectif de masse du solvant contenu dans la solution aqueuse de collagène pour obtenir la formation de la solution concentrée de collagène homogène pur dans la cellule de dialyse ou à la surface de la membrane de dialyse,
d) récupération du moule dans lequel est contenu le matériau ou de la membrane à la surface de laquelle est fixé le matériau de collagène pur homogène.

Au sens de la présente invention on entend par solution de collagène pur une solution ne contenant aucun composé ajouté uniquement dans le but de renforcer les propriétés mécaniques finales du matériau ; il est notamment exempt d'agents réticulants comme les aldéhydes et de polymères de renforts synthétiques comme des polyesters ou naturelles comme le chitosane. En revanche des additifs comme des molécules d'intérêt tels que des agents permettant de modifier sa porosité ou les charges de surfaces du collagène peuvent être ajoutées au matériau.

Le collagène utilisé dans la présente invention peut être d'origine naturelle ou recombinante. Il peut provenir notamment de la peau ou des tendons d'où il est extrait par voie acide ou enzymatique selon des techniques connues de l'homme du métier.

Au sens de l'invention, la cellule de dialyse dont au moins une des extrémités est fermée par une membrane de dialyse, peut également être un moule entièrement constitué en membrane de dialyse. Dans le cas où on injecte une grosse quantité de manière à remplir le moule, la matrice de collagène se fera sur toute la surface du moule pas seulement sur la membrane placée à l'extrémité. Ainsi la forme de l'élément de dialyse va imposer la forme finale du matériau s'il est rempli entièrement. Le produit peut donc être formulé en fonction de l'épaisseur et de la concentration sous forme de film, membrane souple, tube ou moulé à façon.

Les solutions acides de collagène utilisées à l'étape a) sont préparées par des techniques connues de l'homme du métier à partir de collagène naturel ou recombinante. Le solvant aqueux, avantageusement de l'acide acétique à une concentration comprise entre 17 et 500 mM peut contenir différents additifs comme par exemple des sels inorganiques, d'autres types de collagène ou des glycosaminoglycanes tels que l'héparine sulfatée.

Dans un mode de réalisation avantageux de l'invention, la concentration en collagène de la solution initiale utilisée à l'étape a) est comprise entre 0,01 et 5 mg/ml, avantageusement comprise entre 0,5 et 3 mg/ml.

Conformément à l'invention, le polymère utilisé peut être tout polymère hydrosoluble en milieu acide dont le poids moléculaire est supérieur à la taille des pores de l'élément perméable. Il est notamment choisi dans le groupe comprenant le Dextran® et le polyéthylène glycol (PEG). Avantageusement le polymère est du polyéthylèneglycol dont le poids moléculaire est supérieur à celui du collagène donc supérieur à 3000 Da.

La concentration de la solution de polymère peut varier selon les cas et son ajustement est à la portée de l'homme du métier. Si le volume de solution de polymère est très grand alors le volume de solvant provenant de la solution de collagène est négligeable et dilue de façon négligeable la solution de polymère. Dans ce cas, la concentration en polymère initiale sera égale à la concentration en collagène finale. Si le volume de solution de polymère n'est pas grand et la concentration en polymère initiale égale à la concentration en collagène finale, pour ne pas ralentir la diffusion la solution de polymère sera changée régulièrement. Si le volume de solution de polymère n'est pas grand et que la concentration en polymère initiale est supérieure à la concentration en collagène finale, alors la concentration en polymère initiale est calculée de telle façon qu'après dilution par le volume de solvant provenant de la solution de collagène, elle soit égale à la concentration finale voulue en collagène.

### IV- FIBRILLOGENESE DES SOLUTIONS CONCENTREES

Conformément à l'invention, le procédé peut en outre comprendre avant ou après l'étape d), une étape de formation de fibrilles dans le matériau de collagène pur. La formation de ces fibrilles permet de mimer la structure fibrillaire du collagène des tissus biologiques.

La formation des fibrilles est réalisée par toute technique connue de l'homme du métier, notamment par neutralisation de la solution à base de collagène pur homogène par une phase gazeuse (basique) ou liquide (basique ou neutre). Elle peut se faire soit in situ en remplaçant le polymère par la phase gazeuse ou liquide, ce qui permet une préparation « one-pot » du collagène fibrillaire, soit par immersion du matériau de collagène dans une phase gazeuse ou liquide.

Le procédé de l'invention peut également être mis en oeuvre pour fabriquer des produits multi-couches par interruption de l'injection. L'injection peut être interrompue pour atteindre l'équilibre plus vite et reprise afin de faire différentes couches non continues en concentration. Il est également possible d'arrêter l'injection et de ne pas attendre l'équilibre pour avoir différentes couches continues en concentration.

L'invention a également pour objet un matériau homogène à base de collagène pur susceptible d'être obtenu par un procédé tel que défini précédemment.

Le produit obtenu selon l'invention est facilement manipulable sans déchirures et suturable sans réticulation supplémentaire. Il est homogène et ne présente pas de variation de concentrations.

Elles ne déclenchent pas de réaction inflammatoire au niveau du site d'implantation et s'intègrent dans les tissus.

Conformément à l'invention on obtient des matériaux dont la concentration est d'environ 5 à environ 1000 mg/mL, en particulier 5, 10, 20, 30, 40 et 250 mg/mL. Pour une concentration de 5 mg/mL, le module élastique est d'environ 974±239 Pa et le module visqueux d'environ 145±40 Pa. Pour une concentration de 10 mg/mL, le module élastique est d'environ 1287±158 Pa et le module visqueux d'environ 124±12 Pa. Pour une concentration de 20 mg/mL, le module élastique est d'environ 2809±336 Pa et le module visqueux d'environ 263±48 Pa. Pour une concentration de 40 mg/mL, le module élastique est d'environ 9254±1032 Pa et le module visqueux d'environ 786±46 Pa. Les valeurs des modules élastiques et visqueux sont mesurées à 1 Hz et 2 % de déformation.

Selon la concentration en collagène et l'épaisseur du matériau selon l'invention, il peut donc être utilisé comme substitut tissulaire, notamment comme renfort de paroi, pour la fabrication de prothèses, comme substitut de tissu mou ou comme matériau de comblement.

L'invention a donc également pour objet des dispositifs médicaux implantables caractérisés en ce qu'ils comprennent un matériau selon l'invention.

L'invention sera mieux comprise à la lumière des exemples 1 à 3 et des figures 1 à 12 qui l'illustrent.
La figure 1 représente A : une solution de collagène acido-soluble à une concentration inférieure à 5 mg/mL; B : solution concentrée de collagène après dialyse ; C : solution concentrée de collagène selon le procédé de l'invention.
La figure 2 représente un collagène (acido-soluble) (concentration finale -200 mg/mL) obtenu par dialyse qui est macroscopiquement inhomogène (figure 2A). Après neutralisation, la taille des fibres observées en microscopie électronique à balayage est inhomogène (figures 2B à 2D). La concentration initiale de collagène acido-soluble est d'environ 3 mg/mL.
La figure 3 montre un exemple de dispositif utilisé selon l'invention. Dans le cas 1, le volume entier de la cellule de dialyse est rempli et concentré de façon homogène, dans le cas 2, l'injection est arrêtée pendant un temps T afin que l'ensemble de la solution présente dans la cellule se concentre de façon homogène.
La figure 4 représente un collagène (acido-soluble) (concentration finale -250 mg/mL) obtenu selon la technique de l'invention qui est macroscopiquement homogène (figure 4A). Après neutralisation, la taille des fibres observée en microscopie électronique à balayage est homogène (figures 4B à 4D) et s'organise (géométrie cholestérique) avec une homogénéité de structure sur une grande échelle (figure B). La concentration initiale de collagène acido-soluble est d'environ 1 mg/mL.
La figure 5 illustre l'implantation des matrices denses à 300 mg/mL en intramusculaire (A) et en sous-cutanée (B).
La figure 6 illustre l'aspect macroscopique de matrices denses à 20 mg/mL (MD 20 figure 6A) et 40 mg/mL (MD 40 figure 6B) 15 jours après l'implantation intramusculaire.
La figure 7 représente l'aspect macroscopique après 15 et 30 jours d'implantation d'une matrice dense à 300 mg/mL (MD 300).
La figure 8 représente l'aspect microscopique de matrices denses à 20 mg/mL (MD 20 figure 8A) et 40 mg/mL (MD 40 figure 8B) 15 jours après l'implantation en sous-cutané.
La figure 9 représente l'aspect microscopique après 15 jours d'implantation d'une matrice dense à 300 mg/mL (MD300) en sous-cutané à un grossissement x 4. Les flèches représentent les cellules qui migrent/colonisent à l'intérieur du gel
La figure 10 représente l'aspect microscopique après 15 jours d'implantation d'une matrice dense à 300 mg/mL (MD300) en sous-cutané à un grossissement x 10. Les flèches représentent les cellules qui migrent à l'intérieur du gel
La figure 11 représente l'aspect microscopique après 15 jours d'implantation d'une matrice dense à 300 mg/mL (MD300) en intramusculaire à un grossissement x 4. Les flèches représentent les cellules qui migrent à l'intérieur du gel
La figure 12 représente l'aspect microscopique après 15 jours d'implantation d'une matrice dense à 300 mg/mL (MD300) en intramusculaire à un grossissement x 10. Les flèches représentent les cellules qui migrent à l'intérieur du gel.
La figure 13 représente schématiquement le principe de l'obtention de matrices denses par évaporation.
La figure 14 représente schématiquement le principe de l'obtention de matrices denses par dialyse.
La figure 15 représente schématiquement le principe de l'obtention de matrices denses dans des microcellules.
La figure 16 représente schématiquement le principe de l'obtention de matrices denses par compression.

### EXEMPLE 1 : PREPARATION DU MATERIAU SELON L'INVENTION ET CARACTERISATION

### 1.1. Préparation des solutions de collagène initiales

Un collagène de type I est préparé à partir de queues de jeunes rats Wistar, selon le mode opératoire suivant. Les tendons de queue de rat sont excisés dans une hotte à flux laminaire stérile, puis lavés dans une solution tampon phosphate saline contenant 137 mM de NaCl, 2,68 mM de KCl, 8,07 mM de Na₂HPO₄ et 1,47 mM de NaH₂PO₄, pour éliminer les cellules et les traces de sang. Les tendons sont ensuite trempés dans une solution de NaCl 4 M pour éliminer les cellules intactes restantes et précipiter une partie des protéines à poids moléculaire élevé. Après un nouveau lavage avec la solution tampon saline, les tendons sont dissous dans une solution aqueuse à 500 mM d'acide acétique. La solution ainsi obtenue est clarifiée par centrifugation à 41000 g pendant 2 h. Les protéines autres que le collagène de type I sont précipitées de manière sélective dans une solution aqueuse de NaCl 300 mM, puis éliminées par centrifugation à 41000 g pendant 3 h. Le collagène est récupéré à partir du surnageant par précipitation dans une solution aqueuse de NaCl 600 mM suivie d'une centrifugation à 3000 g pendant 45 min. Les culots ainsi obtenus sont dissous dans une solution aqueuse d'acide acétique 500 mM, puis dialysés soigneusement dans le même solvant pour éliminer totalement NaCl. Les solutions sont maintenues à 4°C et centrifugées à 41000 g pendant 4 h avant d'être utilisées.

### 1.2. Préparation du matériau homogène

18 ml d'une solution de collagène à une concentration de 3,2 mg/ml est introduite dans un élément de dialyse muni d'un pousse-seringue électrique tel que décrit à la figure 1. La vitesse d'injection est fixée à 2 µl/min. La dialyse est réalisée à 19 °C en présence de 150 ml d'une solution à 500 mg/mL de polyéthylène glycol de poids moléculaire 35 kDa dissous dans une solution aqueuse d'acide acétique 500 mM. Le temps d'injection est de 8 jours suivis d'un arrêt de 4 jours pour atteindre l'équilibre. La concentration en collagène de la solution acide telle que déterminée avant la fibrillogenèse par détermination de la quantité d'hydroxyproline est de ∼ 250 mg/mL.

La fibrillogénèse est réalisée par immersion du matériau de collagène sous vapeur d'ammoniaque pendant 24 heures.

### 1.3. Mesure de l'homogénéité du matériau obtenu

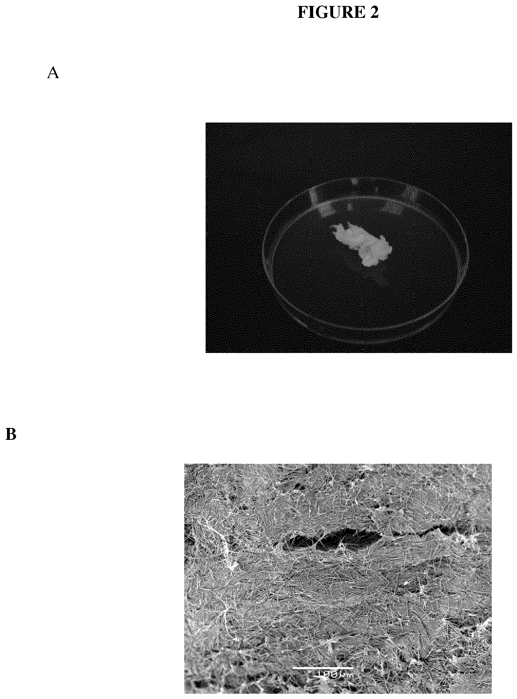

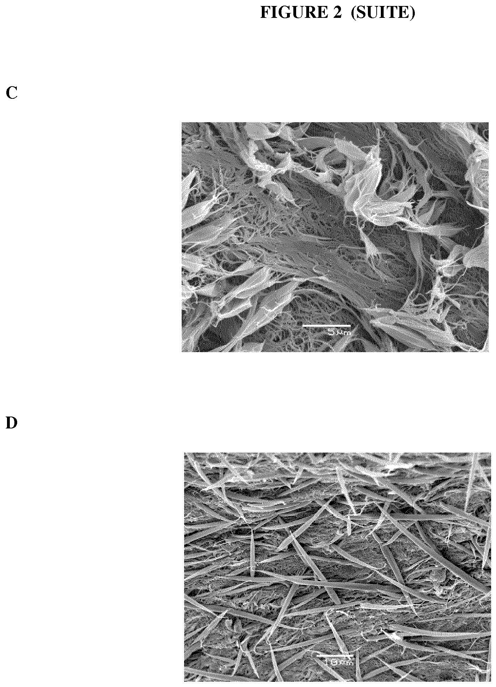

On observe au microscope électronique à balayage des échantillons de matériau obtenu à partir d'une solution ayant une concentration initiale de collagène acido-solubles ∼ 1 mg/mL (Figure 3), soit selon la technique de dialyse inverse classique, soit selon le procédé de l'invention ayant une concentration initiale de collagène acido-solubles ∼ 3 mg/mL. (figure 2).

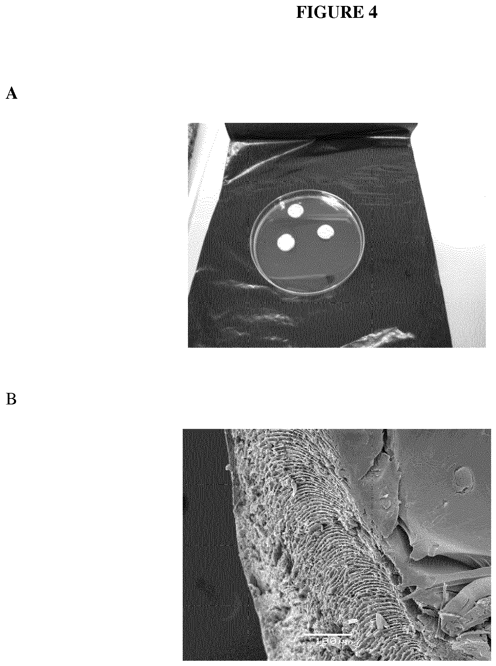

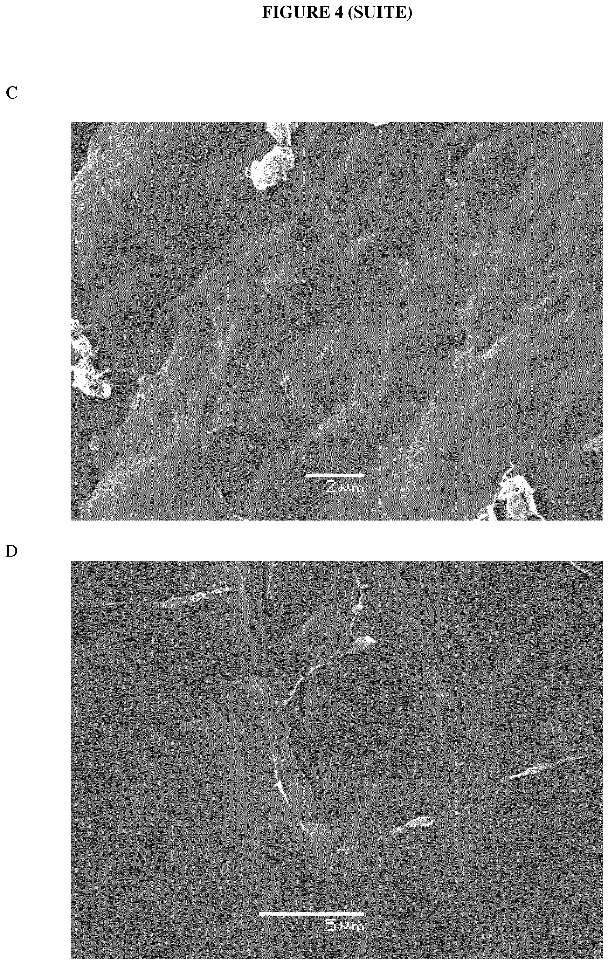

Le collagène (acido-soluble) obtenu par dialyse est macroscopiquement inhomogène (figure 2A) et la taille des fibres est inhomogène (figures 2B à 2D). En revanche le collagène fibrillé obtenu par le procédé selon l'invention est macroscopiquement homogène (figure 4A) et la taille des fibres est également homogène (figures 4B à 4D).

### EXEMPLE 2 : ESSAIS D'IMPLANTATION

### 2.1. Mode opératoire

### 2.1.1. Implantation des matrices denses

Toutes les procédures utilisées sont en accord avec la réglementation en expérimentation animale et les comités d'éthiques de l'INSERM (no. d'agrément 006235, Ministère de l'Agriculture, France).

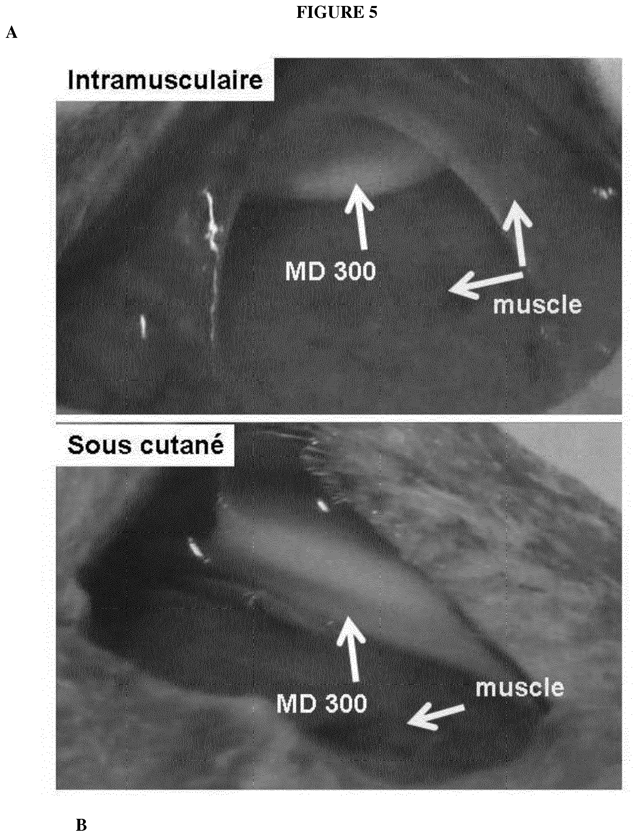

Des rats Wistars de 250g ((Wi/Wi, Charles-Rivers France) sont anesthésiés à l'aide d'une solution de pentobarbital sodique (30 mg/kg, Centravet France). L'abdomen est rasé et désinfecté et une laparotomie est pratiquée sur la ligne médiane. Deux poches sont réalisées de part et d'autre de la ligne médiane : l'une en sous-cutanée, l'autre en intramusculaire. Les matrices denses de collagènes de concentrations variant de 40 à 300 mg/mL sont alors implantées. La poche musculaire est refermée, puis le plan cutané (Vicryl ® 4/0) Voir figure 5.Quinze, trente ou soixante jours après l'implantation, les rats sont euthanasiés à l'aide d'un excès de penthobarbital sodique et les MD sont explantés et fixés en paraformaldehyde à 4% (Merck France). Les échantillons sont ensuite inclus en paraffine.

Les matrices sont facilement manipulables sans déchirures.

### 2.1.2. Analyse histologique

Des coupes sériées de 7 µm sont réalisées puis colorées avec de l'hématoxyline éosine ou du trichrome de Masson. Après observation (microscope Nikon E600 POL) et analyses des sections des photographies sont réalisées (CCD camera, Nikon).

Des études immunohistologiques ont été réalisées sur ces coupes et les différents éléments du remodelage tissulaire ont été mis en évidence (macrophages, témoin de l'inflammation, cellules endothéliales, néovascularisation ou vascularisation).

### 2.2. Résultats

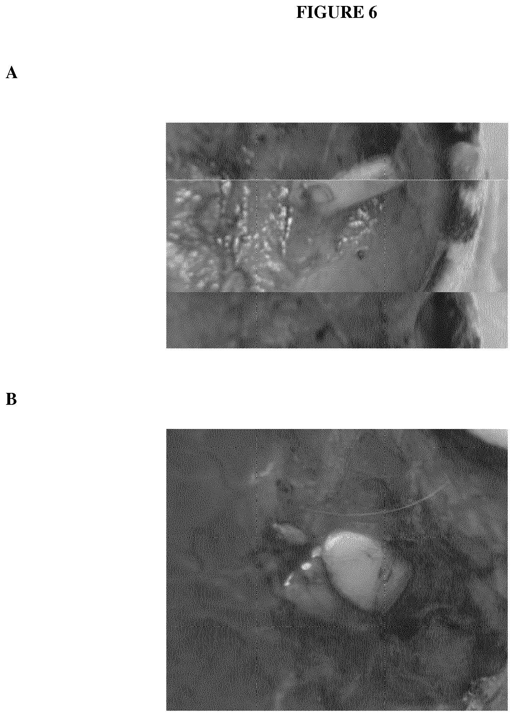

### Ils sont donnés dans les figures 6 à 12

L'implantation des matrices denses ne provoque pas de réaction à corps étrangers sévères après 15 jours d'implantation de matrices à 20 et 40 mg/mL (figures 6A et 6B).

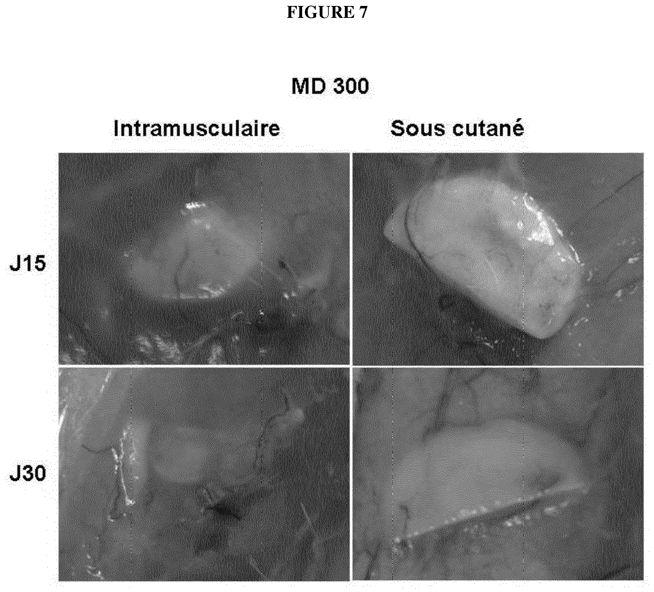

Après 15 et 30 jours d'implantation d'une matrice dense à 300 mg/mL, en sous-cutané la taille de l'échantillon n'a pas varié. En intramusculaire après 30 jours d'implantation le volume de l'échantillon est globalement similaire. Il faudra attendre 60 jours d'implantation pour visualiser une différence (non montré). On note également l'absence de réaction inflammatoire au niveau du site d'implantation d'une matrice à 300 mg/mL (Figure 7).

La vitesse de colonisation des implants par les cellules *in vivo* dépend de la concentration en collagène. Plus la concentration est élevée plus la vitesse est lente. Plus la matrice est épaisse plus la colonisation au coeur du matériau sera longue.

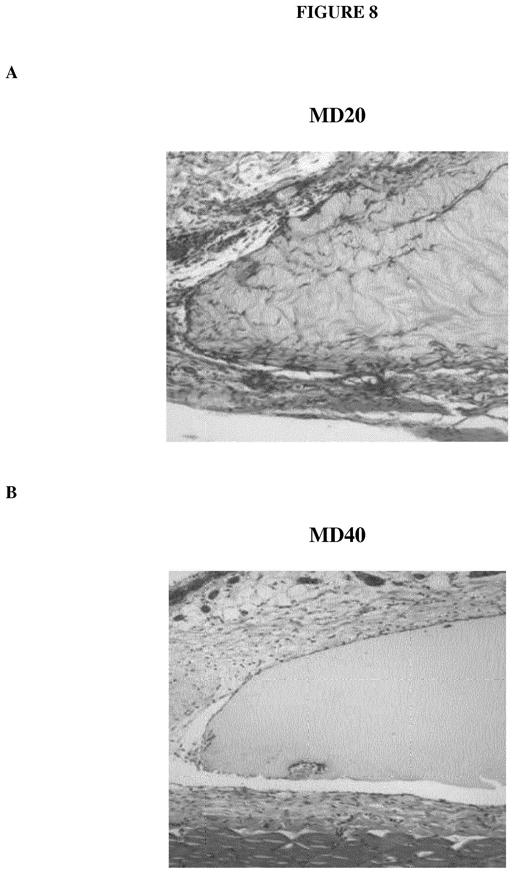

Après 15 jours d'implantation, la matrice dense à 20 mg/mL (MD20) est colonisée par les cellules fibroblastiques (figure 8A). Pour la matrice dense à 40 mg/mL (MD40), au niveau de la berge externe quelques cellules en regard sont observées et commencent à migrer à l'intérieur du gel (Figure 8B).

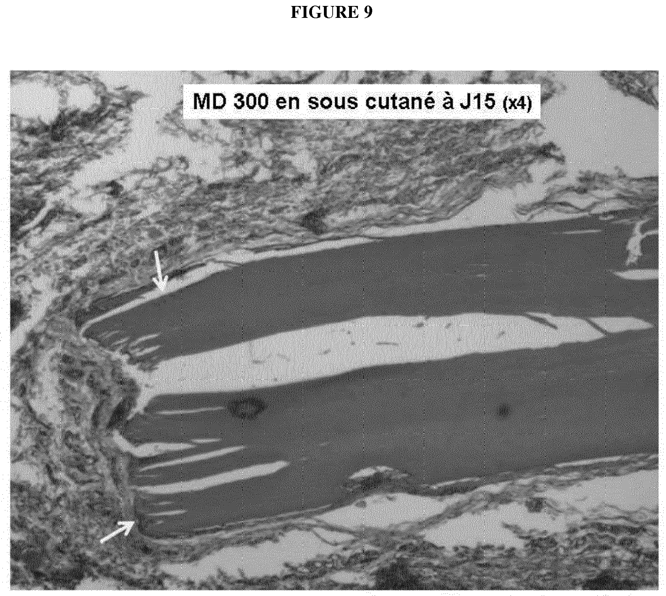

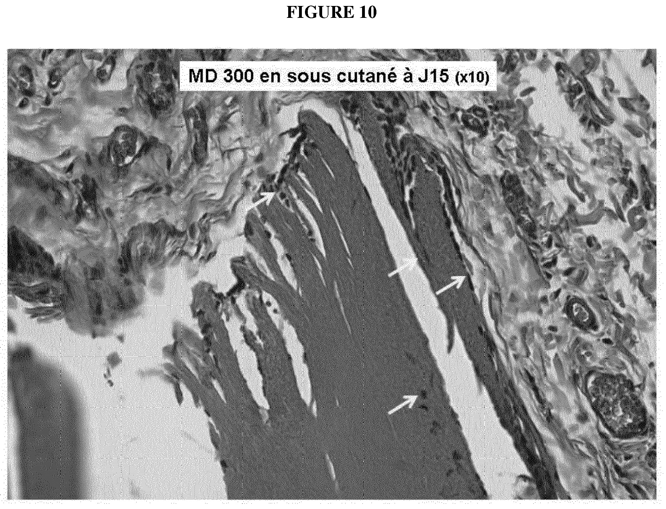

En sous-cutané et d'une manière générale pour les concentrations de 300 mg/mL (MD 300), la colonisation cellulaire se fait plus vers la face cutanée que vers le fascia musculaire. Le MD300 est entouré d'une fine capsule. Au niveau de la berge externe quelques cellules en regard sont observées et commencent à migrer à l'intérieur du gel (figures 9 et 10).

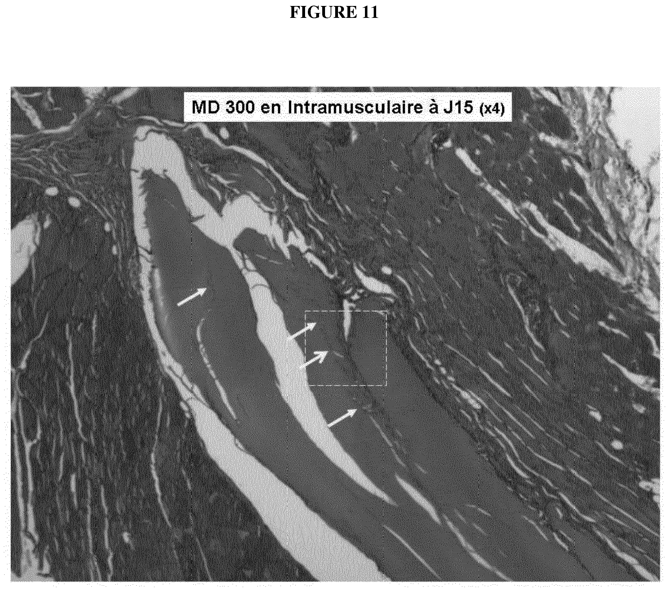

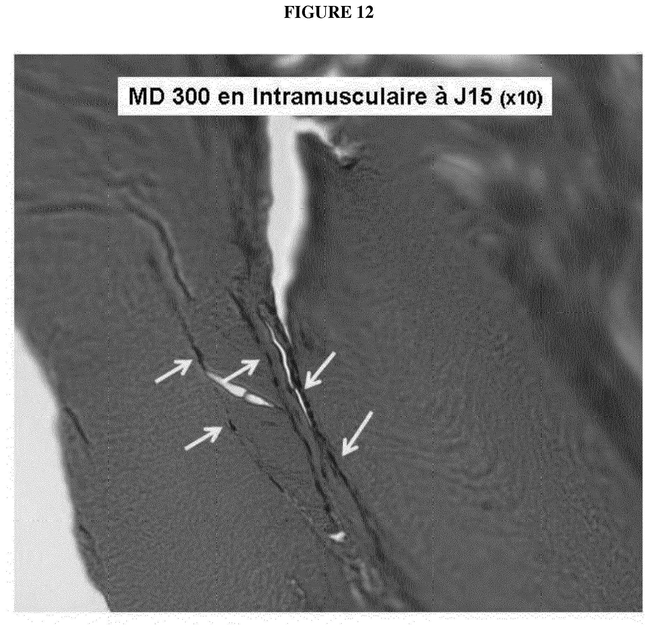

Le décalage entre les résultats après implantation en sous-cutané et intramusculaire est normal. La cicatrisation en intramusculaire est plus rapide du fait de la vascularisation locale (figures 11 et 12).

Les applications peuvent donc être extrêmement variables en fonction du type de matrice dense sélectionnée et du site d'implantation.

## Revendications

1. Procédé de préparation d'un matériau homogène à base de collagène par concentration d'une solution de collagène, ledit procédé comprenant :
a) la mise en contact, par injection continue d'une solution de collagène par des moyens de pression contrôlée, avec un élément perméable, ledit élément perméable étant lui-même en contact avec un agent concentrant a savoir une solution de polymère, l'élément perméable étant choisi avec une taille de pores de poids moléculaire inférieur à celui du collagène d'une part et du polymère externe d'autre part, et le polymère utilisé est un polymère hydrosoluble en milieu acide dont le poids moléculaire est supérieur à la taille des pores de l'élément perméable,
b) le maintien en contact de la solution de collagène, de l'élément perméable et de la solution de polymère dans des conditions permettant le transfert sélectif de masse du solvant contenu dans la solution aqueuse de collagène pour obtenir la formation du matériau de collagène homogène à l'intérieur de l'élément perméable ou à la surface de l'élément perméable.

2. Procédé de préparation d'un matériau homogène à base de collagène selon la revendication 1, **caractérisé en ce que** l'élément perméable est une cellule de dialyse, ledit procédé comprenant les étapes suivantes :
a) préparation d'une solution acide de collagène pur dans un solvant aqueux,
b) injection continue de la solution de collagène pur par des moyens de pression contrôlée, dans des cellules de dialyse dont au moins une des extrémités est fermée par une membrane de dialyse dont la porosité est fixée pour que seuls le solvant ainsi que l'acide et les ions, s'ils sont présents, diffusent à travers ladite membrane, ladite membrane étant en contact avec une solution de polymère, dont la concentration est adaptée à la concentration finale en collagène,
c) maintien de la solution de collagène, de la cellule de dialyse et du polymère dans des conditions permettant le transfert sélectif de masse du solvant contenu dans la solution aqueuse de collagène pour obtenir la formation de la solution concentrée de collagène homogène pur dans la cellule de dialyse ou à la surface de la membrane de dialyse,
d) récupération du moule dans lequel est contenu le matériau ou de la membrane à la surface de laquelle est fixé le matériau de collagène pur homogène.

3. Procédé selon la revendication 1 **caractérisé en ce qu'**il comprend en outre avant ou après l'étape d), une étape de formation de fibrilles dans le matériau de collagène pur.

4. Procédé selon l'une des revendications 1 ou 2 **caractérisé en ce que** la concentration de la solution utilisée à l'étape a) est comprise entre 0,01 et 5 mg/mL, avantageusement comprise entre 0,5 et 3 mg/mL.

5. Procédé selon l'une des revendications 2 ou 4 **caractérisé en ce que** les ions sont des précurseurs d'une phase minérale choisis dans le groupe comprenant les sels de calcium et le phosphate.

6. Procédé selon l'une des revendications précédentes, **caractérisé en ce que** le polymère est choisi dans le groupe comprenant le Dextran® et le polyéthylène glycol.

7. Procédé selon l'une quelconque des revendications précédentes **caractérisé en ce que** le polymère est du polyéthylèneglycol dont le poids moléculaire est supérieur à 3000 Da.

8. Procédé selon l'une quelconque des revendications 3, 6 ou 7 **caractérisé en ce que** les fibrilles sont formées par traitement du matériau à base de collagène pur homogène par une phase gazeuse basique ou liquide neutre ou basique.

9. Matériau homogène, notamment fibrillaire, à base de collagène pur susceptible d'être obtenu par un procédé selon l'une quelconque des revendications 1 à 8.

10. Matériau fibrillaire homogène à base de collagène pur selon la revendication 9 **caractérisé en ce qu'**il contient une ou plusieurs molécules d'intérêt choisies dans le groupe comprenant des collagènes de différents type, des ions, des précurseurs d'une phase minérale, notamment les sels de calcium ou le phosphate, des glycosaminoglycanes et des molécules organiques.

11. Matériau selon la revendication 9 **caractérisé en ce qu'**il se présente sous la forme d'un film, d'une membrane souple, d'un tube ou **en ce qu'**il est moulé à façon.

12. Utilisation du matériau selon l'une quelconque des revendications 9 et 10 comme substitut tissulaire.

13. Dispositifs médicaux implantables **caractérisés en ce qu'**ils comprennent un matériau selon la revendication 9.

## Patentansprüche

1. Verfahren zur Herstellung eines homogenen Materials auf Collagenbasis durch Konzentrieren einer Collagenlösung, wobei das Verfahren umfasst:
a) Inkontaktbringen einer Collagenlösung mit einem durchlässigen Element durch kontinuierliche Injektion durch Mittel mit kontrolliertem Druck, wobei das durchlässige Element selbst in Kontakt mit einem Konzentrationsmittel, nämlich einer Polymerlösung, ist, wobei das durchlässige Element mit einer Porengröße mit einem Molekulargewicht gewählt wird, das unter jenem von Collagen einerseits und des externen Polymers andererseits liegt, wobei das verwendete Polymer ein in saurem Milieu wasserlösliches Polymer ist, dessen Molekulargewicht größer als die Porengröße des durchlässigen Elements ist,
b) Inkontakthalten der Collagenlösung, des durchlässigen Elements und der Polymerlösung unter Bedingungen, die den selektiven Massetransfer des in der wässrigen Collagenlösung enthaltenen Lösemittels erlauben, um die Bildung des homogenen Collagenmaterials im Inneren des durchlässigen Elements oder an der Oberfläche des durchlässigen Elements zu erzielen.

2. Verfahren zur Herstellung eines homogenen Materials auf Collagenbasis nach Anspruch 1, **dadurch gekennzeichnet, dass** das durchlässige Element eine Dialysezelle ist, wobei das Verfahren die folgenden Schritte umfasst:
a) Herstellen einer sauren Lösung von reinem Collagen in einem wässrigen Lösemittel,
b) kontinuierliche Injektion der Lösung von reinem Collagen durch Mittel mit kontrolliertem Druck in die Dialysezellen, wobei wenigstens eines ihrer Enden durch eine Dialysemembran verschlossen ist, deren Porosität derart festgelegt ist, dass nur das Lösemittel sowie die Säure und die Ionen, falls sie vorhanden sind, durch die Membran diffundieren, wobei die Membran mit einer Polymerlösung in Kontakt ist, deren Konzentration an die endgültige Collagenkonzentration angepasst ist,
c) Halten der Collagenlösung, der Dialysezelle und des Polymers unter Bedingungen, die den selektiven Massetransfer des in der wässrigen Collagenlösung enthaltenen Lösemittels erlauben, um die Bildung der konzentrierten Lösung aus reinem homogenem Collagen im Inneren der Dialysezelle oder an der Oberfläche der Dialysemembran zu erzielen,
d) Rückgewinnung der Form, in der das Material enthalten ist, oder der Membran, an deren Oberfläche das homogene Material aus reinem Collagen anhaftet.

3. Verfahren nach Anspruch 1, **dadurch gekennzeichnet, dass** es darüber hinaus vor oder nach dem Schritt d) einen Schritt des Bildens von Fibrillen in dem Material aus reinem Collagen umfasst.

4. Verfahren nach einem der Ansprüche 1 oder 2, **dadurch gekennzeichnet, dass** die Konzentration der im Schritt a) verwendeten Lösung zwischen 0,01 und 5 mg/mL, vorteilhafterweise zwischen 0,5 und 3 mg/mL liegt.

5. Verfahren nach einem der Ansprüche 2 oder 4, **dadurch gekennzeichnet, dass** es sich bei den Ionen um Vorläufer einer mineralischen Phase handelt, die aus der Gruppe ausgewählt sind, die Calciumsalze und Phosphat umfasst.

6. Verfahren nach einem der vorhergehenden Ansprüche, **dadurch gekennzeichnet, dass** das Polymer aus der Gruppe ausgewählt ist, die Dextran^{®} und Polyethylenglycol umfasst.

7. Verfahren nach einem der vorhergehenden Ansprüche, **dadurch gekennzeichnet, dass** es sich bei dem Polymer um Polyethylenglycol handelt, dessen Molekulargewicht größer als 3000 Da ist.

8. Verfahren nach einem der Ansprüche 3, 6 oder 7, **dadurch gekennzeichnet, dass** die Fibrillen durch Behandlung des Materials auf Basis von reinem homogenem Collagen durch eine basische gasförmige Phase oder eine neutrale oder basische Flüssigkeit gebildet werden.

9. Homogenes, insbesondere fibrilläres Material auf Basis von reinem Collagen, das durch ein Verfahren nach einem der Ansprüche 1 bis 8 gewonnen werden kann.

10. Homogenes fibrilläres Material auf Basis von reinem Collagen nach Anspruch 9, **dadurch gekennzeichnet, dass** es ein oder mehrere Moleküle von Interesse enthält, die aus der Gruppe ausgewählt sind, die Collagene verschiedener Typen, Ionen, Vorläufer einer mineralischen Phase, insbesondere Calciumsalze oder Phosphat, Glycosaminoglycane und organische Moleküle umfasst.

11. Material nach Anspruch 9, **dadurch gekennzeichnet, dass** es in Form eines Films, einer biegsamen Membran, eines Rohrs vorliegt oder dass es formgegossen ist.

12. Verwendung des Materials nach einem der Ansprüche 9 und 10 als Gewebeersatz.

13. Implantierbare medizinische Vorrichtungen, **dadurch gekennzeichnet, dass** sie ein Material nach Anspruch 9 umfassen.

## Claims

1. Method for preparing a homogeneous collagen-based material by concentration of a collagen solution, said method comprising:
a) bringing into contact, by continuous injection of a collagen solution by controlled pressure means with a permeable element, said permeable element itself being in contact with a concentrating agent namely a polymer solution, said permeable element being chosen with a pore size of molecular weight less than that of the collagen on the one hand and of the external polymer on the other hand, and the polymer used being a water-soluble polymer in acid medium, the molecular weight of which being greater than the size of the pores of the permeable element,
b) keeping the collagen solution, permeable element and polymer solution in contact under conditions allowing the selective mass transfer of the solvent contained in the aqueous collagen solution in order to obtain the formation of the homogeneous collagen material inside the permeable element or on the surface of the permeable element.

2. Method for preparing a homogeneous collagen-based material according to claim 1 **characterized in that** the permeable element is a dialysis cell, said method comprising the following stages:
a) preparation of an acid solution of pure collagen in an aqueous solvent,
b) continuous injection of the pure collagen solution by controlled pressure means, into dialysis cells at least one of the ends of which is closed by a dialysis membrane the porosity of which is fixed so that only the solvent as well as the acid and the ions, if they are present, diffuse through said membrane, said membrane being in contact with a polymer solution, the concentration of which is adapted to the final collagen concentration,
c) keeping the collagen solution, dialysis cell and polymer under conditions allowing the selective mass transfer of the solvent contained in the aqueous collagen solution in order to obtain the formation of the pure homogeneous concentrated collagen solution in the dialysis cell or on the surface of the dialysis membrane,
d) recovery of the mould in which the material is contained or of the membrane on the surface of which the homogeneous pure collagen material is fixed.

3. Method according to claim 1 **characterized in that**, before or after stage d), it also comprises a stage of formation of fibrils in the pure collagen material.

4. Method according to claim 1 or claim 2 **characterized in that** the concentration of the solution used in stage a) is comprised between 0.01 and 5 mg/mL, advantageously comprised between 0.5 and 3 mg/mL.

5. Method according to claims 2 or 4 **characterized in that** the ions are mineral phase precursors chosen from the group comprising calcium and phosphate salts.

6. Method according to any one of the previous claims **characterized in that** the polymer is chosen from the group comprising Dextran® and polyethylene glycol.

7. Method according to any one of the previous claims **characterized in that** the polymer is polyethylene glycol the molecular weight of which is greater than 3000 Da.

8. Method according to any one of claims 3, 6 or 7 **characterized in that** the fibrils are formed by treatment of the material based on homogeneous pure collagen by a basic gas, or neutral or basic liquid phase.

9. Homogeneous material, notably fibrillar, based on pure collagen capable of being obtained by a method according to any one of claims 1 to 8.

10. Homogeneous fibrillar material based on pure collagen according to claim 9 **characterized in that** it contains one or more molecules of interest chosen from the group comprising collagens of different types, ions, mineral phase precursors, notably calcium or phosphate salts, glycosaminoglycans and organic molecules.

11. Material according to claim 9 **characterized in that** it is presented in the form of a film, a flexible membrane, a tube or **in that** it is moulded for the purpose.

12. Use of the material according to any one of claims 9 and 10 as tissue substitute.

13. Implantable medical devices **characterized in that** they comprise a material according to claim 9.
